Europäisches Patentamt

European Patent Office

Office européen des brevets

(11) Numéro de publication: **0 235 715 B1**

## FASCICULE DE BREVET EUROPEEN

(45) Date de publication de fascicule du brevet:
**15.05.91**

(51) Int. Cl.⁵: **C07C 49/557**, **A24B 15/34**,
**A23L 1/226**

(21) Numéro de dépôt: **87102520.1**

(22) Date de dépôt: **23.02.87**

(54) **Cétone tricyclique et son utilisation à titre d'ingrédient aromatisant.**

(30) Priorité: **03.03.86 CH 856/86**

(43) Date de publication de la demande:
**09.09.87 Bulletin 87/37**

(45) Mention de la délivrance du brevet:
**15.05.91 Bulletin 91/20**

(84) Etats contractants désignés:
**CH DE FR GB LI NL**

(56) Documents cités:
**FR-A- 2 175 236**

**TETRAHEDRON, vol. 42, no. 12, 1986, pages 3311-3321, Pergamon Journals Ltd. Oxford, GB; A.F. THOMAS et al.:
"4-(1-Hydro-peroxy-1-methylethyl)1,3-cyclo-pentadienyl methyl ketone: its formation from alpha-terpineol and behaviour as a di-methylfulvene epxide"**

(73) Titulaire: **FIRMENICH SA**
**1, route des Jeunes Case Postale 239**
**CH-1211 Genève 8(CH)**

(72) Inventeur: **Thomas, Alan Francis**
**Chemin de Grens**
**CH-1261 Borex(CH)**
Inventeur: **Smith, Alistair Yorke**
**20, avenue de l'Amandolier**
**Ch-1208 Geneve(CH)**

(74) Mandataire: **Salvadori, Giuseppe, Dr.**
**c/o Firmenich S.A. Case Postale 239**
**CH-1211 Genève 8(CH)**

EP 0 235 715 B1

## Description

La présente invention a pour objet une cétone tricyclique nouvelle de formule (I) ou 3,10-diacétyl-11-isopropylidène-7,7-diméthyl-tricyclo [6.2.1.0 $^{2,6}$]undéca-2,5,9-triène ainsi que son utilisation à titre d'ingrédient aromatisant. Elle a en outre pour objet un procédé pour renforcer, améliorer ou modifier l'arôme de la fumée formée lors de la combustion de tabac, procédé qui consiste à incorporer à une base, constituée par un tabac naturel ou un produit de remplacement du tabac, d'origine naturelle ou synthétique, ladite cétone tricyclique de formule(I) en tant qu'ingrédient aromatisant actif.

L'invention est définie comme indiqué aux revendications.

Il est bien connu que le tabac utilisé pour la fabrication des cigarettes est essentiellement composé d'un mélange de différents types de tabacs qui donnent à la fumée l'arôme particulier recherché par le consommateur. Ainsi, les cigarettes ordinaires contiennent des mélanges de tabacs de Virginie, du Maryland ou du Kentucky et des tabacs orientaux ou turcs, les proportions respectives variant selon l'arôme desdits mélanges de tabac.

Il est de pratique courante dans l'industrie de modifier l'arôme du tabac au moyen d'ingrédients aromatisants qui en rehaussent, arrondissent ou adoucissent le goût propre. C'est ainsi que les produits à fumer peuvent être enrichis en des parties moins nobles de la feuille de tabac, telles les nervures, lesquelles parties sont généralement caractérisées par un goût âcre.

La présente invention apporte une solution nouvelle au problème posé par l'aromatisation du tabac. Nous avons en effet découvert que la cétone tricyclique(I) possédait des propriétés organoleptiques utiles et qu'elle pouvait de ce fait trouver un emploi avantageux en tant qu'ingrédient aromatisant, tout particulièrement pour l'aromatisation d'articles à fumer, spécialement à base de tabac.

Le 3,10-diacétyl-11-isopropylidène-7,7-diméthyl-tricyclo[6.2.1.0 $^{2,6}$]undéca-2,5,9-triène est un composé nouveau. Il peut être obtenu grâce à un procédé original à partir d'alpha-terpinéol selon le schéma réactionnel en annexe.

La transformation d'alpha-terpinéol en 1-(4-isopropylidène-1-cyclopent-1-ényl)-1-éthanone(II) s'effectue conformément à la méthode décrite par G.Bozzato et al., J. Chem. Soc., Chem. Commun. (1974), 1005, au moyen d'une réaction d'ozonolyse en milieu méthanolique, suivie d'un traitement à l'acide phosphorique.

Laissé simplement à l'air et à l'abri de la lumière, le produit obtenu se transforme en l'hydropéroxyde-(III), ou [4-(1-hydropéroxy-1-méthyléthyl)-1,3-cyclo-pentadién-1-yl]-1-éthanone. Ce dernier composé est ensuite réduit en la cétone tricyclique désirée au moyen d'un agent de réduction ordinaire. A cet effet, un iodure de métal alcalin ou, de préférence, une phosphine aromatique, telle la triphénylphosphine, conviennent parfaitement.

La cétone(I) de l'invention peut être employée à titre d'ingrédient aromatisant isolé ou, de préférence, en mélange avec d'autres ingrédients usuels, aussi bien d'origine naturelle que synthétique. L'homme de l'art connaît par expérience l'éventail d'ingrédients aromatiques utiles et il n'est pas nécessaire ici d'en définir la nature spécifique. Des exemples particuliers ont par ailleurs été donnés dans des ouvrages spécialisés et, à cet effet, on peut mentionner ceux décrits par S. Arctander, "Perfume and Flavor Chemicals", Montclair, N.J. (1969); G. Fenaroli, "Handbook of Flavor Ingredients", 2ème éd., CRC Press, Inc., Cleveland, Ohio (1975); S.van Straten et H. Maarse, "Volatile Compounds in Food", Institut CIVO-Analysis TNO (1983).

Parmi les coingrédients aromatisants destinés plus particulièrement à l'aromatisation du tabac, il convient de mentionner les ingrédien ts décrits dans le brevet francais 2'175'236.

Les proportions dans lesquelles le composé de l'invention peut être employé varient dans une gamme de valeurs assez étendue. Ces proportions varient selon l'effet organoleptique recherché et selon la provenance du tabac auquel le composé est ajouté. Des effets aromatisants intéressants peuvent être obtenus avec des quantités de l'ordre de 0,1 à 10ppm (parties par million), quantités basées sur le poids de la matière aromatisée. De préférence, leur valeur se situe à environ 0,1-1,0 ppm. Dans tous les cas, les limites données ci-dessus ne sauraient être considérées comme absolues.

Le composé de l'invention rappelle l'odeur de feuilles, voire de la paille et sert à développer des notes vertes, légèrement florales, métalliques, astringentes, grasses, légèrement amères et terreuses. Son goût, en application dans un tabac passe presque inaperçu. Ce n'est que lors de l'activation qui s'accompagne à la combustion du tabac que son arôme se développe au mieux; ce qui suggère que le produit se décompose sous l'effet de la chaleur. Il y a tout lieu de penser par ailleurs que sa décomposition donne naissance à du 2-acétyl-6,6-diméthylfulvène de formule (IV) lequel composé serait éminemment responsable de l'arôme conféré à la fumée.

Instable dans des conditions ordinaires de température et pression, le 2-acétyl-6,6-diméthylfulvène dimérise pour se transformer en la cétone(I). Ce phénomène est à considérer comme positif pour l'emploi

2

envisagé dans la présente invention. En effet, dans l'aromatisation du tabac, l'on recherche souvent des ingrédients actifs organoleptiquement neutres, voire inodores à température ambiante, qui peuvent toutefois dégager un arôme pendant le processus de combustion.

L'invention est illustrée de manière plus détaillée par les exemples suivants dans lesquels les températures sont indiquées en degrés centigrades et les abréviations ont le sens usuel dans l'art.

Exemple 1

Préparation de 3,10-diacétyl-11-isopropylidène-7,7-diméthyl-tricyclo [6.2.1.0[2,6]]undéca-2,5,9-triène

a. 76g de 1-(4-isopropylidène-1-cyclopent-1-ényl)-1-éthanone, obtenus à partir de 154g d'alpha-terpinéol selon G.Bozzato et al., J.Chem. Soc., Chem. Commun. (1974), 1005, cristallisés dans le pentane, ont été laissés exposés à l'air et à l'abri de la lumière pendant 3 jours. Le produit a été ensuite recristallisé dans l'éther et a fourni le [4-(1-hydropéroxy-1-méthyléthyl)-1,3-cyclopentadién-1-yl]-1-éthanone ayant F. 82-84°.

```
UV : lambda_max 222 nm (epsilon 6830), 300 nm (epsilon 2120) ;
IR : 3525, 1690 cm^-1 ;
1H-RMN (CDCl3) : 1,51 (6H, s) ; 2,37 (3H, s) ; 3,42 (2H, d,
    J=1,5 cps) ; 6,51 et 7,29 (chacun 1H) delta ppm ;
13C-RMN : q à 25,2, 26,0 ; t à 39,5 ; d à 128,0, 143,4 ; s à 81,4,
    146,1, 161,7, 194,8 ;
SM : (M+-H2O) : 164(7) ; m/z : 149(100), 121(40), 93(32), 77(27),
    43(30) ;
    Trouvé :  C 66,2%  ;  calculé pour C10H14O3    C 65,9%
              H  7,9%                              H  7,7%
```

b. 5,7g de triphénylphosphine ont été ajoutés par petites portions à une solution de 4g de l'hydropéroxyde obtenu sous lettre a. ci-dessus dans 20ml de chloroforme. Pendant l'addition, la température de la solution a été maintenue en dessous de 25° par refroidissement extérieur. Après 1h, la solution a été concentrée et le résidu résultant a été chromatographié sur une colonne de gel de silice (éluant: éther-pentane 1:1). Après élution de 6,4g d'oxyde de triphénylphosphine, on a élué 1,5g de la cétone désirée ayant F. 138-9° après recristallisation dans l'éther.

```
UV : lambda_max 238 nm (epsilon 10500), 305 nm (epsilon 6700) ;
IR : 1632, 1663 cm^-1 ;
1H-RMN : 1,24 ; 1,25 ; 1,70 ; 1,75 ; 2,27 ; 2,69 (chacun 3H, s) ;
    système ABX avec A 3,12 ; B 3,20 ; X 6,29 (JAB 22 ; JAX et
    JBX 2,5) ; 3,21 (1H, d, J=3 cps) ; 5,16 (1H, s) ; 7,08 (1H, d,
    J=3) delta ppm.
```

Une élution ultérieure de la colonne de gel de silice a permis l'isolation de 1,5g de 3,10-diacétyl-11-isopropylidène-7,7-diméthyl-tricyclo [6.2.1.0[2,6]]undéca-3,5,9-triène dont le spectre de masse était identique à celui de l'isomère 2,5,9-triène décrit plus haut.

SM : 148(57), 133(100), 105(50), 103(20), 79(28), 77(30), 63(12), 51(14), 43(13), 39(10).

Par traitement avec de l'alumine basique (Fluka AG, Buchs, activité 1, g. 0,5), 0,5g de l'isomère 3,5,9-triène dans 10 ml d'éther ont été transformés en l'isomère 2,5,9-triène.

Exemple 2

Le 3,10-diacétyl-11-isopropylidène-7,7-diméthyl-tricyclo[6.2.1.0$^{2,6}$] undéca-2,5,9-triène, obtenu selon le procédé décrit à l'Exemple 1, a été injecté à raison de 0,1, respectivement 1 ppm, dans deux échantillons de cigarettes commerciales (marque: Marlboro, Philip Morris Co.) sous forme de solution dans l'alcool éthylique à 95%. Après avoir été laissées au repos pendant 48h, les cigarettes ainsi aromatisées ont été soumises à une évaluation organoleptique de la part d'un groupe d'experts. Leur goût et leur arôme ont été déterminés par comparaison avec des cigarettes identiques non aromatisées.

Aux deux dosages indiqués, la fumée des cigarettes aromatisées présentait un arôme plus arrondi avec une note moins acidulée. La fumée avait nettement un caractère aromatique plus complet. Au dosage le plus élevé, la fumée était plus douce avec une nuance caramel et érable. L'arôme des cigarettes avant combustion était par contre inchangé.

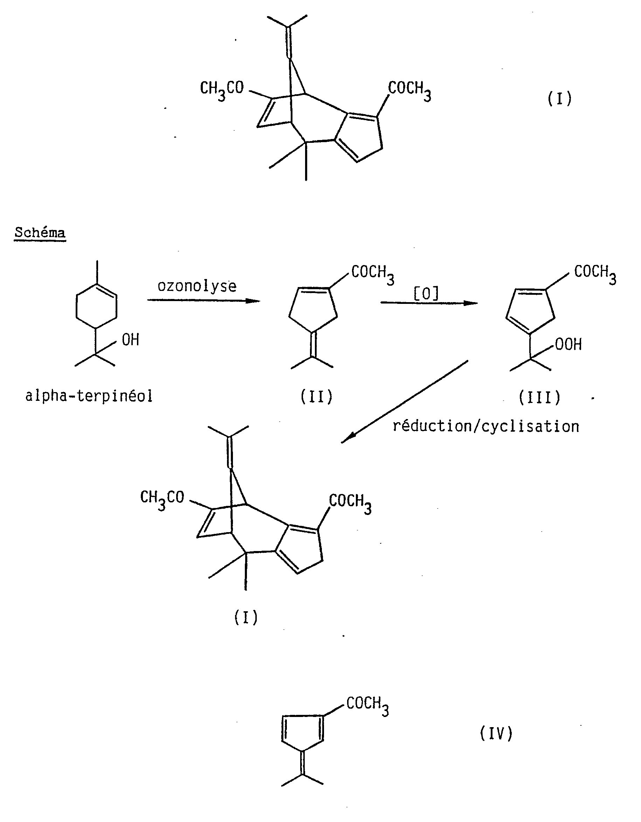

**Revendications**

1. 3,10-Diacétyl-11-isopropylidène-7,7-diméthyl-tricyclo[6.2.1.0$^{2,6}$]undéca-2,5,9-triène.

2. Utilisation du composé selon la revendication 1, à titre d'ingrédient aromatisant pour l'aromatisation d'articles à fumer.

3. Articles à fumer résultant de l'utilisation selon la revendication 2.

4. Articles à fumer selon la revendication 3, à base de tabac naturel ou artificiel.

5. Procédé pour renforcer, améliorer ou modifier l'arôme de la fumée formée lors de la combustion de tabac caractérisé en ce qu'on incorpore à une base, constituée par un tabac naturel ou un produit de remplacement du tabac, d'origine naturelle ou synthétique, une quantité organoleptiquement active du composé selon la revendication 1.

6. Procédé pour la préparation du composé selon la revendication 1, caractérisé en ce qu'on
   a. oxyde à l'air la 1-(4-isopropylidène-1-cyclopent-1-ényl)-1-éthanone pour fournir la [4-(1-hydropéroxy-1-méthyléthyl)-1,3-cyclopentadién-1-yl]-1-éthanone, et
   b. réduit et cyclise le composé ainsi obtenu au moyen d'un agent réducteur.

7. Procédé selon la revendication 6, caractérisé en ce que l'on utilise comme agent réducteur la triphénylphosphine.

**Claims**

1. 3,10-Diacetyl-11-isopropylidene-7,7-dimethyl-tricyclo[6.2.1.0$^{2,6}$] undeca-2,5,9-triene.

2. Use of the compound according to claim 1, as an aroma ingredient for the flavouring of smoking articles.

3. Smoking articles resulting from the use according to claim 2.

4. Smoking articles according to claim 3, based on natural or artificial tobacco.

5. Method to enhance, improve or modify the aroma of the smoke formed by the combustion of tobacco characterized in that an organoleptically effective amount of the compound according to claim 1 is incorporated into a base constituted by a tobacco of natural origin or by a tobacco substituting product, of natural or synthetic origin.

6. Process for the preparation of the compound according to claim 1 characterized by :
   a. the air-oxidation of 1-(4-isopropylidene-1-cyclopent-1-enyl)-1-ethanone to give [4-(1-hydroperoxy-1-methylethyl)-1,3-cyclopentadien-1-yl]-1-ethanone, and
   b. the reduction and cyclisation of the resulting compound by means of a reducing agent.

7. A process according to claim 6 characterized in that the reducing agent is triphenylphosphine.

**Ansprüche**

1. 3,10-Diacetyl-11-isopropyliden-7,7-dimethyl-tricyclo[6.2.1.0$^{2,6}$] undeca-2,5,9-trien.

2. Verwendung der Verbindung gemäß Anspruch 1, als Geschmackstoffzusatz zur Aromatisierung von rauchbaren Artikeln.

3. Rauchbare Artikeln resultierend aus der Verwendung gemäß Anspruch 2.

4. Rauchbare Artikeln gemäß Anspruch 3, die aus natürlichem oder synthetischem Tabak bestehen.

5. Verfahren zur Verstärkerung, Verbesserung oder Veränderung des Aromas des aus der Tabakverbrennung gebildeten Rauchs, dadurch gekennzeichnet, daß man zu einer natürlichen oder synthetischen Base, die aus natürlichem Tabak oder aus einem Tabaksersatzprodukt besteht, eine wirksame Menge der Verbindung gemäß Anspruch 1 gibt.

6. Verfahren zur Herstellung der Verbindung gemäß Anspruch 1, dadurch gekennzeichnet, daß man :
   a. 1-(4-Isopropyliden-1-cyclopent-1-enyl)-1-ethanon luftoxydiert, um [4-(1-Hydroperoxy-1-methylethyl)-1,3-cyclopentadien-1-yl]-1-ethanon zu erhalten, und
   b. die so erhaltene Verbindung mittels eines Reduktionsmittel reduziert und cyclisiert.

7. Verfahren gemäß Anspruch 6, dadurch gekennzeichnet, daß man, als Reduktionsmittel, Triphenylphosphin verwendet.

(I)

Schéma

alpha-terpinéol      ozonolyse → (II)    [O] → (III)

réduction/cyclisation

(I)

(IV)